**Europäisches Patentamt**

**European Patent Office**

**Office européen des brevets**

(19)

(11) Veröffentlichungsnummer : **0 370 200 B1**

(12)

# EUROPÄISCHE PATENTSCHRIFT

(45) Veröffentlichungstag der Patentschrift :
**09.12.92 Patentblatt 92/50**

(51) Int. Cl.$^5$ : **C07C 15/08,** C07C 7/14

(21) Anmeldenummer : **89117728.9**

(22) Anmeldetag : **26.09.89**

(54) **Verfahren zur Gewinnung von p-Xylol mit einer Reinheit von mehr als 99,8 Gew.-%.**

(30) Priorität : **19.11.88 DE 3839229**

(43) Veröffentlichungstag der Anmeldung :
**30.05.90 Patentblatt 90/22**

(45) Bekanntmachung des Hinweises auf die
Patenterteilung :
**09.12.92 Patentblatt 92/50**

(84) Benannte Vertragsstaaten :
**DE FR GB**

(56) Entgegenhaltungen :
**WO-A-88/05766**

(73) Patentinhaber : **Krupp Koppers GmbH
Altendorfer Strasse 120
W-4300 Essen 1 (DE)**

(72) Erfinder : **Puppel, Günter
Kampstrasse 4
W-4273 Wulfen (DE)**

EP 0 370 200 B1

**Beschreibung**

Die Erfindung betrifft ein Verfahren zur Gewinnung von p-Xylol mit einer Reinheit von mehr als 99,8 Gew.-% aus einem durch Kristallisation gewonnenen Vorprodukt, dessen Reinheit bei ca. 98 Gew.-% liegt.

Es ist bekannt, für die Abtrennung des p-Xylols aus den als Ausgangsmaterial dienenden $C_8$-Aromaten, die neben den drei Xylolisomeren noch Ethylbenzol enthalten können, Kristallisationsverfahren einzusetzen. Hierbei wird von der Tatsache Gebrauch gemacht, daß die Schmelzpunkte der einzelnen $C_8$-Isomere des Xylols ausgeprägte Temperaturdifferenzen aufweisen. Mit den heute üblichen Kristallisationsverfahren gelingt es jedoch nur mit erheblichem Aufwand, als Endprodukt ein p-Xylol zu erzeugen, dessen Reinheit über 99,5 Gew.-% liegt. Dabei wird p-Xylol, das in einer Zentrifuge ohne zusätzliche Behandlung mit einer Reinheit von ca. 98 Gew.-% anfallen würde, in der Zentrifuge mit Endprodukt oder hierfür geeigneten Medien (wie z.B. Toluol), die sich destillativ vom p-Xylol wieder abtrennen lassen, auf die gewünschte Endreinheit von über 99,5 Gew.-% gebracht.

In neuester Zeit ist es jedoch einigen Anbietern unter Anwendung eines besonderen Adsorptionsverfahren gelungen, ein p-Xylol mit einer Reinheit von über 99,8 Gew.-% auf den Markt zu bringen, was natürlich eine entsprechende Nachfrage nach diesem sehr reinen Produkt verursacht hat.

Mit Hilfe von Kristallisationsverfahren war man jedoch bisher nur unter großem Aufwand und Produktionsverlust in der Lage, ein p-Xylol mit vergleichbar hoher Reinheit herzustellen, so daß sich für die Betreiber derartiger, nach dem Kristallisationsverfahren arbeitender Anlagen ein echter Wettbewerbsnachteil ergeben hat.

Der Erfindung liegt deshalb die Aufgabe zugrunde, ein Verfahren zur Gewinnung von p-Xylol mit einer Reinheit von mehr als 99,8 Gew.-% zu schaffen, das von einem durch Kristallisation gewonnenen Vorprodukt ausgeht, dessen Reinheit bei ca. 98 Gew.-% liegt.

Das der Lösung dieser Aufgabe dienende Verfahren ist erfindungsgemäß dadurch gekennzeichnet, daß folgende Verfahrensschritte zur Anwendung gelangen:

a) Das kristalline Vorprodukt wird in einem Mischbehälter mit vorgekühltem Wasser sowie aus der Stufe e) des Verfahrens zurückgeführtem p-Xylol, dessen Reinheit bei ca. 96 Gew.-& liegt, mit einer Temperatur von 0 bis 13°C, vorzugsweise 5 bis 7°C, durchmischt;

b) das sich im Mischbehälter einstellende p-Xylol-Kristall-Wasser-Gemisch wird, sobald der Kristallanteil des Gemisches ca. 30 Gew.-% beträgt, kontinuierlich über ein Vorentwässerungsfilter, in dem eine Eindickung bis auf 70 - 80 Gew.-% Kristallanteil erfolgt, in eine nachgeschaltete Reinigungszentrifuge abgezogen;

c) in der Reinigungszentrifuge wird in der ersten Stufe die flüssige Phase weitgehend von den p-Xylolkristallen abgetrennt, worauf der hierbei anfallende p-Xylolkristallbrei in der zweiten Stufe mit einem Teilstrom des Endproduktes versetzt, auf ca. 13°C erwärmt und anschließend von der noch anhaftenden flüssigen Phase befreit wird, wobei die dabei anfallenden p-Xylolkristalle in einen Schmelzbehälter abgezogen werden;

d) die im Schmelzbehälter aufgeschmolzenen p-Xylolkristalle werden als Endprodukt mit der gewünschten Reinheit abgezogen, wobei der Teilstrom des Endproduktes, der in die zweite Stufe der Reinigungszentrifuge zurückgeführt wird, zuvor auf eine Temperatur von 60 bis 80°C erwärmt wird,

und

e) die in der zweiten Stufe der Reinigungszentrifuge von den p-Xylolkristallen abgetrennte flüssige Phase wird in einen Scheidebehälter eingeleitet, in dem das p-Xylol abgetrennt und daran anschließend in die Stufe a) des Verfahrens zurückgeführt wird, während die im Scheidebehälter anfallende wäßrige Phase, die aus einer Wasser-Xylol-Emulsion besteht, in einem nachgeschalteten Koaleszer in ihre Bestandteile zerlegt wird, die getrennt voneinander ebenfalls in die Stufe a) des Verfahrens zurückgeführt werden.

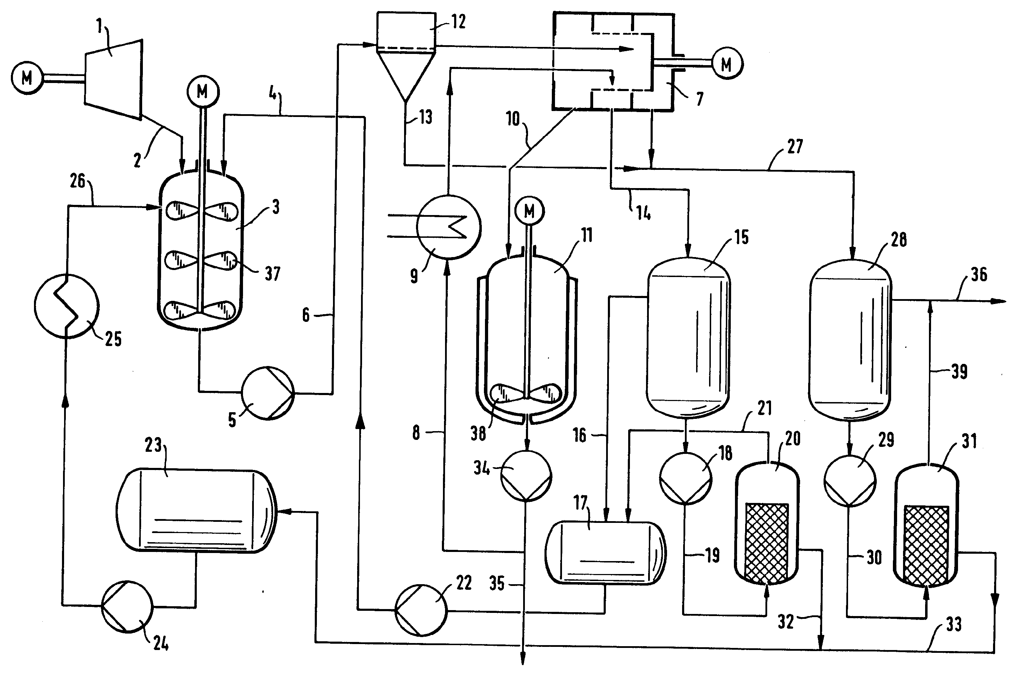

Weitere Einzelheiten des erfindungsgemäßen Verfahrens ergeben sich aus den vorliegenden Unteransprüchen und sollen nachfolgend anhand des in der Abbildung dargestellten Fließschemas erläutert werden.

Das als Vorprodukt dienende kristallförmige p-Xylol wird mit einer Reinheit von ca. 98 Gew.-% aus der Zentrifuge 1 abgezogen und mit einer Temperatur von -5 bis 7°C über die Leitung 2 in den Mischbehälter 3 eingeleitet. Dieses Vorprodukt kann dabei nach einem der zum Stande der Technik gehörenden Kristallisationsverfahren gewonnen werden, da mit diesen Verfahren eine Reinheit von ca. 98 Gew.-% ohne spezielle Behandlung des Kristallbreis erreicht werden kann. Die aus der Zentrifuge 1 stammenden p-Xylolkristalle weisen noch eine gewisse Restfeuchte auf, die bei ca. 3 - 6 Gew.-% liegt.

Gleichzeitig mit den p-Xylolkristallen wird über die Leitung 4 das aus dem Behälter 17 stammende p-Xylol, dessen Reinheit bei ca. 96 Gew.-% liegt, in den Mischbehälter 3 eingeleitet. Auf die Gewinnung dieses p-Xylols wird weiter unten noch näher eingegangen werden. Durch die kontinuierliche Zufuhr von Wasser über die Leitung 26 bei gleichzeitigem Abzug des sich bildenden p-Xylolkristall-Wasser-Gemisches durch die Pumpe 5 wird die Temperatur im Mischbehälter 3 im Bereich zwischen 0 und 13°C, vorzugsweise zwischen 5 und 7°C, konstant gehalten.

Der Mischbehälter 3 ist mit einem Rührer 37 versehen, um das teilweise entstandene, nach oben treibende Kristallagglomerat in Mischung zu halten.

Nach einer Verweilzeit von ca. 30 Sekunden wird das p-Xylolkristall-Wasser-Gemisch durch die Pumpe 5 über die Leitung 6 zum Vorentwässerungsfilter 12 gefördert. Dieses Gemisch weist einen Kristallanteil von ca. 30 Gew.-% auf. Im Vorentwässerungsfilter 12 wird soviel Flüssigkeit entzogen, daß eine Eindickung bis auf einen Kristallanteil von 70 - 80 Gew.-% erfolgt. Die flüssige Phase wird über Leitung 13 und 27 zum Scheidebehälter 28 abgeleitet, während der Kristallbrei in die Reinigungszentrifuge 7 gefördert wird.

In der Reinigungszentrifuge 7 wird das p-Xylolkristall-Wasser-Gemisch zunächst in der ersten Stufe weiter entwässert. Die dabei anfallende flüssige Phase wird über die Leitung 27 in den Scheidebehälter 28 abgezogen, während der resultierende p-Xylolkristallbrei mit einem Teilstrom des Endproduktes versetzt wird. Dieser Teilstrom, der über die Leitung 8 in die Reinigungszentrifuge 7 zurückgeführt und zuvor im Wärmeaustauscher 9 bis auf eine Temperatur von 60 bis 80°C erwärmt wird, bewirkt eine Erwärmung der vorgetrockneten p-Xylolkristalle und eine Vermischung mit der an den Kristallen noch anhaftenden flüssigen Phase. Die auf diese Weise bis auf eine Temperatur von ca. 13°C erwärmten und nachgetrockneten p-Xylolkristalle verlassen mit hoher Reinheit über den Schacht 10 die Reinigungszentrifuge 7 und gelangen in den beheizten Schmelzbehälter 11, der zur Verbeserung des Wärmeüberganges mit dem Rührer 38 versehen sein kann.

Die von den p-Xylolkristallen abzentrifugierte Flüssigkeit wird währenddessen über die Leitung 14 in den Scheidebehälter 15 abgezogen. Im Schmelzbehälter 11 werden die p-Xylolkristalle aufgeschmolzen und dann durch die Pumpe 34 über die Leitung 35 als Endprodukt mit der gewünschten Reinheit von mehr als 99,8 Gew.-% zum nicht dargestellten Lagerbehälter gepumpt. Von der Leitung 35 zweigt die Leitung 8 ab, durch die der Teilstrom des Endproduktes abgezogen wird, der in die Reinigungszentrifuge 7 zurückgeführt wird. Die Größe dieses Teilstromes liegt zwischen 6 und 20 Gew.-% des insgesamt über die Leitung 35 abgezogenen Endproduktes.

Die im Scheidebehälter 15 gesammelte Flüssigkeit wird durch Phasenscheidung in eine Xylolphase und eine wäßrige Phase getrennt. Die Xylolphase wird dabei trennschichtgeregelt über die Leitung 16 zum Behälter 17 abgezogen, während die wäßrige Phase als schwere Phase durch die Pumpe 18 über die Leitung 19 in den Koaleszer 20 gefördert wird. Hier wird diese Phase, die aus einer Wasser-Xylol-Emulsion besteht, in ihre Bestandteile zerlegt. Das Xylol wird über die Leitung 21 abgezogen und mit dem im Behälter 17 befindlichen Xylol vereinigt, während das Wasser über die Leitung 32 in die Leitung 33 eingeleitet wird. Aus dem Behälter 17 wird das Xylol, dessen Reinheit bei ca. 96 Gew.-% liegt, durch die Pumpe 22 über die Leitung 4 in den Mischbehälter 3 zurückgefördert.

Die über die Leitung 27 in den Scheidebehälter 28 abgeleitete Flüssigkeit wird in diesem ebenfalls durch Phasenscheidung in eine Xylolphase und eine wäßrige Phase zerlegt. Die Xylolphase wird hierbei über die Leitung 36 abgezogen. Da ihr p-Xylolgehalt bei ca. 85 - 87 Gew.-% liegt, kann sie in den Kristallisationsprozeß vor der Zentrifuge 1 zurückgeführt werden. Die wäßrige Phase besteht wiederum aus einer Wasser-Xylol-Emulsion und wird mittels der Pumpe 29 über die Leitung 30 in den Koaleszer 31 gepumpt, in dem die Trennung in die beiden Bestandteile erfolgt.

Das abgetrennte p-Xylol wird über die Leitung 39 dem p-Xylol in der Leitung 36 zugemischt. Das anfallende Wasser fließt aus dem Koaleszer 31 über die Leitung 33 in den Behälter 23. Von dort wird das Wasser durch die Pumpe 24 über die Leitung 26 in den Mischbehälter 3 zurückgepumpt, nachdem es im Wärmetauscher 25 bis auf eine Temperatur von 2 bis 10°C gekühlt worden ist.

Abschließend soll die Wirksamkeit des erfindungsgemäßen Verfahrens noch durch ein Ausführungsbeispiel belegt werden. Hierbei wurde das als Einsatzprodukt dienende p-Xylol aus der Zentrifuge 1 mit einer Reinheit von 98 Gew.-% und einer Restfeuchte von ca. 6 % in den Mischbehälter 3 eingeleitet. Die Temperatur des p-Xylols lag dabei zwischen - 2 und 0°C, während vorgekühltes Wasser mit einer Temperatur von ca. 9°C in den Mischbehälter 3 eingeführt wurde. Gleichzeitig wurde über die Leitung 4 p-Xylol mit einer Reinheit von 96,6 Gew.-% und einer Temperatur von 12°C zugemischt. Pro kg Einsatzprodukt wurden hierbei 0,1 kg 96,6 %iges Xylol zugemischt. Mit einem Kristallanteil von ca. 30 Gew.-% und einer Temperatur von ca. 7,5°C wurde das resultierende p-Xylolkristall-Wasser-Gemisch aus dem Mischbehälter 3 abgezogen und in den Vorentwässerungsfilter 12 eingeleitet, in dem eine Voreindickung bis auf einen Kristallanteil von 70 Gew.-% erreicht wurde. Anschließend wurde das Gemisch der Reinigungszentrifuge 7 zugeführt, in der die weitere Aufarbeitung, wie weiter oben beschrieben, erfolgte. Das über die Leitung 35 abgezogene, aufgeschmolzene Endprodukt weist eine Reinheit von 99,9 Gew.-% auf. Von diesem Endprodukt wurden ca. 10 Gew.-% über die Leitung 8 abgezweigt und nach Erwärmung auf eine Temperatur von 70°C in die Reinigungszentrifuge 7 zurückgeführt.

**Patentansprüche**

1.　Verfahren zur Gewinnung von p-Xylol mit einer Reinheit von mehr als 99,8 Gew.-% aus einem durch Kristallisation gewonnenen Vorprodukt, dessen Reinheit bei ca. 98 Gew.-% liegt, dadurch gekennzeichnet, daß folgende Verfahrensschritte zur Anwendung gelangen:

a) Das kristalline Vorprodukt wird in einem Mischbehälter mit vorgekühltem Wasser sowie aus der Stufe e) des Verfahrens zurückgeführtem p-Xylol, dessen Reinheit bei ca. 96 Gew.-% liegt, mit einer Temperatur von 0 bis 13°C, vorzugsweise 5 bis 7°C, durchmischt;

b) das sich im Mischbehälter einstellende p-Xylolkristall-Wasser-Gemisch wird, sobald der Kristallanteil des Gemisches ca. 30 Gew.-% beträgt, kontinuierlich über ein Vorentwässerungsfilter, in dem eine Eindickung bis auf 70 - 80 Gew.-% Kristallanteil erfolgt, in eine nachgeschaltete Reinigungszentrifuge abgezogen;

c) in der Reinigungszentrifuge wird in der ersten Stufe die flüssige Phase weitgehend von den p-Xylolkristallen abgetrennt, worauf der hierbei anfallende p-Xylolkristallbrei in der zweiten Stufe mit einem Teilstrom des Endproduktes versetzt, auf ca. 13°C erwärmt und und anschließend von der noch anhaftenden flüssigen Phase befreit wird, wobei die dabei anfallenden p-Xylolkristalle in einen Schmelzbehälter abgezogen werden;

d) die im Schmelzbehälter aufgeschmolzenen p-Xylolkristalle werden als Endprodukt mit der gewünschten Reinheit abgezogen, wobei der Teilstrom des Endproduktes, der in die zweite Stufe der Reinigungszentrifuge zurückgeführt wird, zuvor auf eine Temperatur von 60 bis 80°C erwärmt wird,

und

e) die in der zweiten Stufe der Reinigungszentrifuge von den p-Xylolkristallen abgetrennte flüssige Phase wird in einen Scheidebehälter eingeleitet, in dem das p-Xylol abgetrennt und daran anschließend in die Stufe a) des Verfahrens zurückgeführt wird, während die im Scheidebehälter anfallende wäßrige Phase, die aus einer Wasser-Xylol-Emulsion besteht, in einem nachgeschalteten Koaleszer in ihre Bestandteile zerlegt wird, die getrennt voneinander ebenfalls in die Stufe a) des Verfahrens zurückgeführt werden.

2. Verfahren nach Anspruch 1, dadurch gekennzeichnet, daß die in der Vorentwässerung und in der ersten Stufe der Reinigungszentrifuge abgetrennte flüssige Phase in einen Scheidebehälter eingeleitet wird, in dem das Xylol von der wäßrigen Phase abgetrennt und daran anschließend in den der Herstellung des Vorproduktes dienenden Kristallisationsprozeß zurückgeführt wird, während die wäßrige Phase, die aus einer Wasser-Xylol-Emulsion besteht, in einem nachgeschalteten Koaleszer in ihre Bestandteile zerlegt wird, wobei das dabei anfallende Xylol ebenfalls in den der Herstellung des Vorproduktes dienenden Kristallisationsprozeß und das Wasser in die Stufe a) des Verfahrens zurückgeführt werden.

3. Verfahren nach den Ansprüchen 1 und 2, dadurch gekennzeichnet, daß zwischen 6 und 20 Gew.-% des insgesamt anfallenden Endproduktes in die zweite Stufe der Reinigungszentrifuge zwecks Erwärmung des Kristallbreis zurückgeführt werden.

4. Verfahren nach den Ansprüchen 1 bis 3, dadurch gekennzeichnet, daß die Erwärmung des Kristallbreis in der zweiten Stufe der Reinigungszentrifuge auf ca. 13°C zusätzlich mit erhitzter Umblaseluft der Zentrifuge, Dampf oder aufgesprühtes heißes Wasser erfolgt.

5. Verfahren nach den Ansprüchen 1 bis 4, dadurch gekennzeichnet, daß das Vorprodukt mit einer Temperatur von - 5°C bis 7°C und das Wasser mit einer Temperatur von 2 bis 10°C in den Mischbehälter eingeleitet wird.

**Claims**

1. Process for the recovery of p-xylene having a purity above 99.8% by weight from a preproduct obtained by crystallisation and having a purity of about 98% by weight, characterised in that the following process steps are carried out:

a) the crystalline preproduct is thoroughly mixed in a mixing vessel at a temperature of 0 to 13°C, preferably 5 to 7°C, with precooled water and about 96% by weight pure p-xylene recycled from stage e) of the process,

b) the p-xylene crystal/water mixture forming in the mixing vessel is, as soon as the crystal content of the mixture is about 30% by weight, continuously taken off via a predehydration filter, in which thickening up to 70-80% by weight crystal content takes place, into a downstream purification centrifuge,

c) in the purification centrifuge, the liquid phase is largely separated off from the p-xylene crystals in the first stage, whereupon the p-xylene crystal mass thus arising is treated in the second stage with a part stream of the end product, warmed to about 13°C and then freed of the still adhering liquid phase, the p-xylene crystals thus arising being taken off into a melting vessel,

d) the p-xylene crystals melted in the melting vessel are taken off as end product having the desired purity, the part stream of the end product, which is recycled to the second stage of the purification centrifuge, first being heated to a temperature of 60 to 80°C, and

e) the liquid phase separated off from the p-xylene crystals in the second stage of the purification centrifuge is introduced into a separator vessel, in which the p-xylene is separated off and subsequently thereto recycled to stage a) of the process, while the aqueous phase, which arises in the separator vessel and consists of a water/xylene emulsion, is split in a downstream coalescer into its constituents which are, separately from one another, likewise recycled to stage a) of the process.

2. Process according to Claim 1, characterised in that the liquid phase separated off in the predehydration and in the first stage of the purification centrifuge is introduced into a separator vessel in which the xylene is separated off from the aqueous phase and subsequently thereto recycled to the crystallisation process serving for producing the preproduct, while the aqueous phase, which consists of a water/xylene emulsion, is split into its constituents in a downstream coalescer, the xylene thus arising being likewise recycled to the crystallisation process serving for producing the preproduct and the water being recycled to stage a) of the process.

3. Process according to Claims 1 and 2, characterised in that between 6 and 20% by weight of the total end product arising is recycled to the second stage of the purification centrifuge for the purpose of warming the crystal mass.

4. Process according to Claims 1 to 3; characterised in that the warming of the crystal mass to about 13°C in the second stage of the purification centrifuge is effected additionally with heated circulating air of the centrifuge, steam or sprayed-on hot water.

5. Process according to Claims 1 to 4, characterised in that the preproduct is introduced at a temperature of -5°C to 7°C and the water is introduced at a temperature of 2 to 10°C into the mixing vessel.

**Revendications**

1. Procédé pour l'obtention de p-xylène d'une pureté supérieure à 99,8 % en poids à partir d'un avant-produit qui est obtenu par cristallisation et dont la pureté se situe à 98 % en poids environ,

caractérisé par le fait que l'on utilise les phases opératoires suivantes :

a) Dans un récipient de mélange, on mélange l'avant-produit cristallin avec de l'eau préalablement refroidie, ainsi qu'avec du p-xylène recyclé de l'étape e) du procédé, dont la pureté est d'environ 96 % en poids, à une température de zéro à 13°C, de préférence de 5 à 7°C ;

b) Le mélange de p-xylène cristallin et d'eau s'établissant dans le récipient de mélange, dès que la fraction cristalline du mélange atteint environ 30 % en poids, est soutiré en continu dans une centrifugeuse de purification montée en aval, par un filtre de pré-déshydratation dans lequel a lieu un épaississement jusqu'à obtention d'une fraction cristalline de 70 à 80 % en poids ;

c) Dans la centrifugeuse de purification, on sépare largement dans le premier étage la phase liquide des cristaux de p-xylène, après quoi on ajoute dans le second étage, à la bouillie cristalline de p-xylène qui se forme alors, un courant partiel du produit final, chauffe le tout à 13°C environ, puis le débarrasse ensuite de la phase liquide encore adhérante, en soutirant dans un récipient de fusion les cristaux de p-xylène qui se forment alors ;

d) On soutire comme produit final présentant la pureté désirée les cristaux de p-xylène fondus dans le récipient de fusion, en chauffant préalablement, à une température de 60 à 80°C, le courant partiel du produit final qui est recyclé dans le second étage de la centrifugeuse de purification,

et

e) La phase liquide séparée des cristaux de p-xylène dans le second étage de la centrifugeuse de purification est introduite dans un récipient séparateur dans lequel le p-xylène est séparé et renvoyé ensuite dans l'étage a) du procédé, tandis que la phase aqueuse qui se rassemble dans le récipient séparateur et est constituée par une émulsion d'eau et de xylène, est décomposée en ses constituants dans un coalesceur monté en aval, lesdits constituants étant également recyclés séparément l'un de l'autre dans l'étage a) du procédé.

2. Procédé selon la revendication 1, caractérisé par le fait que la phase liquide séparée lors de la pré-déshydratation et dans le premier étage de la centrifugeuse de purification est introduite dans un récipient séparateur dans lequel le xylène est séparé de la phase aqueuse et est ensuite recyclé dans le processus de cristallisation servant à la préparation de l'avant-produit, tandis que la phase aqueuse, qui est constituée par une émulsion d'eau et de xylène, est décomposée en ses constituants dans un coalesceur monté en aval, tandis que le xylène qui se forme alors est également recyclé dans le processus de cristallisation servant à la préparation de l'avant-produit et que l'eau ést recyclée dans l'étage a) du procédé.

3. Procédé selon les revendications 1 et 2, caractérisé par le fait qu'entre 6 et 20 % en poids du produit final se formant au total sont recyclés dans le second étage de la centrifugeuse de purification en vue d'échauffer la bouillie cristalline.

4. Procédé selon les revendications 1 à 3, caractérisé par le fait que l'échauffement de la bouillie cristalline dans le second étage de la centrifugeuse de purification a lieu à 13°C, complémentairement avec de l'air chauffé de brassage de la centrifugeuse, de la vapeur ou de l'eau chaude pulvérisée.

5. Procédé selon les revendications 1 à 4, caractérisé par le fait que l'avant-produit est introduit à une température de -5°C à + 7°C et que l'eau est introduite à une température de 2 à 10°C dans le récipient de mélange.

EP 0 370 200 B1